(19) Europäisches Patentamt
European Patent Office
Office européen des brevets

(11) **EP 3 062 746 B1**

(12) **EUROPEAN PATENT SPECIFICATION**

(45) Date of publication and mention
of the grant of the patent:
**16.05.2018 Bulletin 2018/20**

(51) Int Cl.:
*A61F 2/30* (2006.01)     *A61F 2/38* (2006.01)
*A61F 2/32* (2006.01)     *A61B 17/80* (2006.01)
*A61B 90/00* (2016.01)

(21) Application number: **14793754.4**

(22) Date of filing: **28.10.2014**

(86) International application number:
**PCT/US2014/062565**

(87) International publication number:
**WO 2015/065969 (07.05.2015 Gazette 2015/18)**

(54) **IMPLANT DESIGN USING HETEROGENEOUS BONE PROPERTIES AND PROBABILISTIC TOOLS TO DETERMINE OPTIMAL GEOMETRIES FOR FIXATION FEATURES**

IMPLANTATDESIGN MIT HETEROGENEN KNOCHENEIGENSCHAFTEN UND PROBABILISTISCHEN WERKZEUGEN ZUR BESTIMMUNG DER OPTIMALEN GEOMETRIEN FÜR FIXIEREINRICHTUNGEN

MODÈLE D'IMPLANT UTILISANT DES PROPRIÉTÉS OSSEUSES HÉTÉROGÈNES ET UNE TECHNIQUE PROBABILISTE POUR DÉTERMINER LES GÉOMÉTRIES OPTIMALES DE CARACTÉRISTIQUES DE FIXATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2013 US 201361896335 P**

(43) Date of publication of application:
**07.09.2016 Bulletin 2016/36**

(73) Proprietor: **Stryker Corporation**
**Kalamazoo, MI 49002 (US)**

(72) Inventors:
• **DAVIGNON, Robert**
**Morris Plains, NJ 07950 (US)**
• **FERKO, Michael C.**
**Warwick, NY 10990 (US)**

(74) Representative: **Valea AB**
**Anna Lindhs Plats 4**
**211 19 Malmö (SE)**

(56) References cited:
**WO-A1-2013/170872     WO-A2-2008/021494**
**KR-A- 20120 092 451     US-A1- 2008 234 833**

**Description**

BACKGROUND OF THE INVENTION

In cementless orthopedic procedures, robust biologic ingrowth is generally a key element to long term implant stability and performance. Biologic ingrowth generally requires sufficient stability of the implant with respect to the adjacent bones and/or tissues particularly during the first 6-8 months after implantation. During this time, bone growth onto a roughened or into a porous surface generally only occurs if the implant is held stably such that the motion of the implant relative to the bone is less than 150 microns.

Implant manufacturers routinely utilize a variety of design features to attempt to provide a press-fit that aids in limiting movement of the implant relative to surrounding anatomical structures. Fixation features such as pegs and keels, for example, generally include designed surface textures to increase the coefficient of friction between the implant and the surrounding anatomical structures. These implant fixation features generally result in increasing success of cementless implants.

However, bone properties, including bone density, porosity, and elastic modulus, for example, vary by location within a patient. Variability in bone properties at the location of fixation features results in variable effect on implant fixation and subsequently can result in decreased implant stability.

Variations in bone properties should therefore be incorporated into the design of implant fixation features in order to, for instance, achieve a reduction in excess micromotion and maintain a desirable range of strain and/or stress transmission. More so, by taking into account variations in bone properties, such as bone density, the fit of the articular implant may be less likely to fail during increased loading scenarios.

Patent application KR2012 0092451 A discloses a method of designing at least one fixation feature of an implant for a bone comprising the features of the preamble of claim 1.

BRIEF SUMMARY OF THE INVENTION

The invention is defined in the independent claim 1. Preferred embodiments are disclosed in the dependent claims.

A first aspect of the present invention is to take advantage of information derived about bone quality in order to achieve an optimized fit between an articular implant and a bone. One characteristic of an optimized fit may include that the engagement between the implant and the bone is less susceptible to failure under various loading scenarios. Another characteristic of an optimized fit may include that the micromotion, stress transmission, and strain of the implant is substantially minimized.

According to one aspect of the disclosure, a method of designing an implant includes obtaining image data corresponding to at least one bone and deriving bone property information from the image data. A feature of the implant may be determined based at least in part on the derived bone property information. The implant may be manufactured to substantially match the determined feature. The image data may be, for example, CT image data. The image data may also correspond to other suitable imaging methods, including magnetic resonance imaging ("MRI"), Electrical Impedance Tomography, Dual-Energy X-ray Absorptiometry, X-ray, ultrasound, and nuclear imaging, for example. The bone may be a bone of a knee or other joint, or any other bone. The image data may corresponds to a single individual, a population of individuals, or a subpopulation of individuals. The step of deriving the bone property information from the image data may include the step of determining at least one of Hounsfield values, bone density, or elastic modulus. The step of determining the feature of the implant may include creating a virtual bone model, mapping the derived bone property information to the virtual bone model, and superimposing a virtual implant model on the bone model in a desired position, the virtual implant model including one or more virtual fixation features characterized by one or more input parameters. The virtual implant model may be loaded with a virtual physiological load, and finite element analysis may be performed to determine value ranges for at least one of the input parameters of the one or more virtual fixation features. The one or more virtual fixation features of the virtual implant model may be modified based on the determined value ranges for the one or more input parameters. The one or more virtual fixation features may include a bone contacting surface, a peg, or a keel, for example. The one or more input parameters may include at least one input parameter selected from the group consisting of peg location, peg depth, peg angle, peg curvature, peg size, press-fit, peg shape, bone contacting geometry, and surgical placement degrees of freedom. The step of modifying the virtual fixation features of the virtual implant model based on the determined value ranges may substantially minimize one or more of micromotion, stress transmission, and strain.

An articular implant for repairing a joint may include a first surface configured to contact a bone of the joint and a second surface opposing the first surface. The implant may include at least one fixation feature for fixing the articular implant to the bone, wherein at least one parameter of the at least one fixation feature has a value determined by simulating a virtual implant acting on a virtual bone model incorporating bone property information. The at least one fixation feature may protrude outwardly from the first surface of the articular implant and/or may have a geometry based at least in part on the derived bone density information. The geometry of the at least one fixation feature may be selected from the group consisting of height, width, length, and radius.

According to the invention, a method of designing at least one fixation feature of an implant for a bone includes creating a virtual model of the bone and mapping bone property information to the virtual bone, the bone property information derived from image data. The method also includes creating a virtual model of the implant including at least one virtual fixation feature characterized by at least one input parameter, and performing a first simulation of an implantation of the virtual implant on the virtual bone with a first value for the at least one input parameter, the first simulation resulting in at least a first value for an output parameter. A second simulation of the implantation of the virtual implant on the virtual bone is performed with a second value for the at least one input parameter, the second simulation resulting in at least a second value for the output parameter. A relationship is derived between the values of the input parameter and the values of the output parameter, and the fixation feature of the implant is designed based on the derived relationship.

The bone property information may be selected from the group of bone density and elastic modulus. The step of deriving a relationship between the values of the input parameters and the values of the output parameter may include creating a contour plot or response surface. The implant may be an articular implant, including a knee implant or a hip implant, or a non-articular implant, including a bone plate. For a knee implant, the fixation feature may be a selected from the group consisting of a peg, a keel, and a bone contacting geometry. The input parameter may be selected from the group consisting of fixation feature location, fixation feature depth, fixation feature angle, fixation feature curvature, fixation feature size, fixation feature press-fit, fixation feature shape, fixation feature bone contacting geometry, and placement of degrees of freedom of the fixation feature. For an acetabular cup component of a hip implant, the fixation feature may be one or more through holes in the articular cup component, and the input parameter may be selected from the group consisting of the number of through holes in the acetabular cup component and a location of the one or more through holes in the acetabular cup component. If the fixation feature is one or more screws configured to be inserted through the acetabular cup component, the input parameter may be selected from the group of screw size and thread property. If the implant is a femoral component of a hip implant, the fixation feature may be a femoral stem of the femoral component, and the input parameter may be selected from the group consisting of femoral stem width, femoral stem length, femoral stem curvature, femoral stem bone contacting geometry, and femoral stem shape.

If the implant is a non-articular implant, such as a bone plate, the fixation feature may be one or more through holes in the bone plate, and the input parameter may be selected from the group consisting of the number of through holes in the bone plate and a location of the one or more through holes in the bone plate. Alternatively, or in addition, the fixation feature may be one or more screws configured to be inserted through the bone plate, and the input parameter may be selected from the group of screw size and thread property.

The output parameter may be selected from the group consisting of micro motion, strain transmission, stress transmission, stress shielding. The virtual implant may be intended for use with cement or other adhesive, and the output parameter may be selected from the group of stress transmission to the cement or other adhesive and strain transmission to the cement or other adhesive.

According to still another embodiment of the disclosure, an implant for a bone may designed according to the methods described above, wherein the implant includes at least one fixation feature. The implant may be, for example, an articular implant or a non-articular implant.

BRIEF DESCRIPTION OF THE DRAWINGS

The present invention will be better understood on reading the following detailed description of non-limiting embodiments thereof, and on examining the accompanying drawings, in which:

FIG. 1 is a side plan view of bone properties mapped to virtual bone of one embodiment of a proximal tibia;
FIG. 2 is a top plan view of the proximal tibia of FIG. 1 with mapped bone density information;
FIG. 3 is a view of one embodiment of a tibial implant having fixation features parametrically modified to determine optimal placement; and
FIG. 4 is a perspective view of one embodiment of a proximal tibia having an implant engaged to a resected portion thereof with the implant loaded under an example of case loading for anterior liftoff.

DETAILED DESCRIPTION

As described above, bone properties may vary by location within a patient, which, if not accounted for, may be a potential cause of loss of implant stability. Variations in bone properties, or quality of bone, should therefore be incorporated into the design of implant fixation features in order to, for instance, achieve a reduction in excess micromotion, maintain a desirable range of strain and/or stress transmission, minimize the change in strain the bone experiences with an implant compared to healthy native bone in order to prevent stress shielding, and/or minimize the stress and/or strain experienced by cement or other adhesives that facilitate fixation to extend the life of the implant and/or cement. These endpoints may serve as outputs in simulations, as described in greater detail below. More so, by taking into account variations in bone properties, such as bone density, the fit of the articular implant may be less likely to fail during increased loading scenarios.

By taking into account heterogeneous bone properties such as bone density, the fixation features of an implant, such as an articular implant, can be designed such that the fit of the implant is optimized when engaged to bone. For example, an optimized fit can be obtained by deriving a bone density model and using finite element analysis in order to determine the ideal fixation feature geometry of an implant. It should be understood that, as used herein, when the terms "optimal" or "optimized" or "ideal" is used to modify another term, the term so modified need not be actually perfect, but rather is used to refer to a desired characteristic.

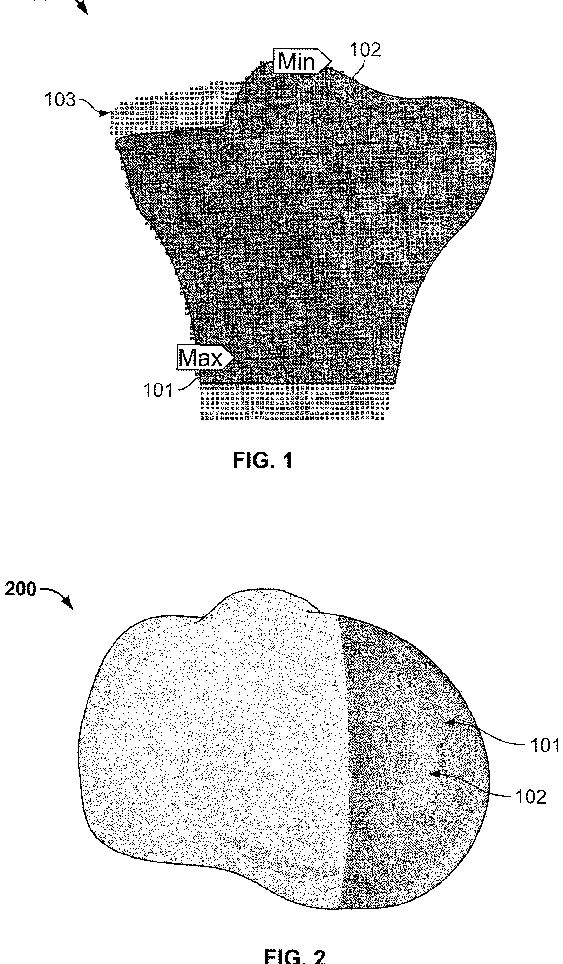

In FIG. 1, one example of a virtual bone model 100 with mapped local bone properties 101 and 102 is presented. In this example, a Computed Tomography ("CT") scan is used to create a virtual 3D bone model 100. Other imaging modalities may be used to create the virtual 3D bone model such as Magnetic Resonance Imaging ("MRI"), for example. Creation of the virtual 3D bone model may include the use of medical imaging software, such as Mimics® software for medical image segmentation, for example. Local bone properties derived from the CT scan are virtually mapped to their corresponding positions on the 3D bone model 100. In FIG. 1, Hounsfield values are determined via the CT scan and then mapped to the 3D bone model 100, which shows areas of increased 101 and decreased 102 CT brightness.

Further, in FIG. 1, the portion of the 3D bone model 103 that corresponds to the portion of bone that would be resected during surgery is discarded. Thus, the corresponding local bone properties mapped to the empty space 103 are ignored.

In FIG. 2, a virtual bone model 200 with mapped bone density properties is presented. The Hounsfield values derived from the CT scan can be correlated to bone density and/or elastic modulus, for example. In this case, Hounsfield values mapped to the virtual 3D bone model are converted into local bone density values. This results in a virtual 3D bone model showing areas of decreased bone density 101 and increased bone density 102.

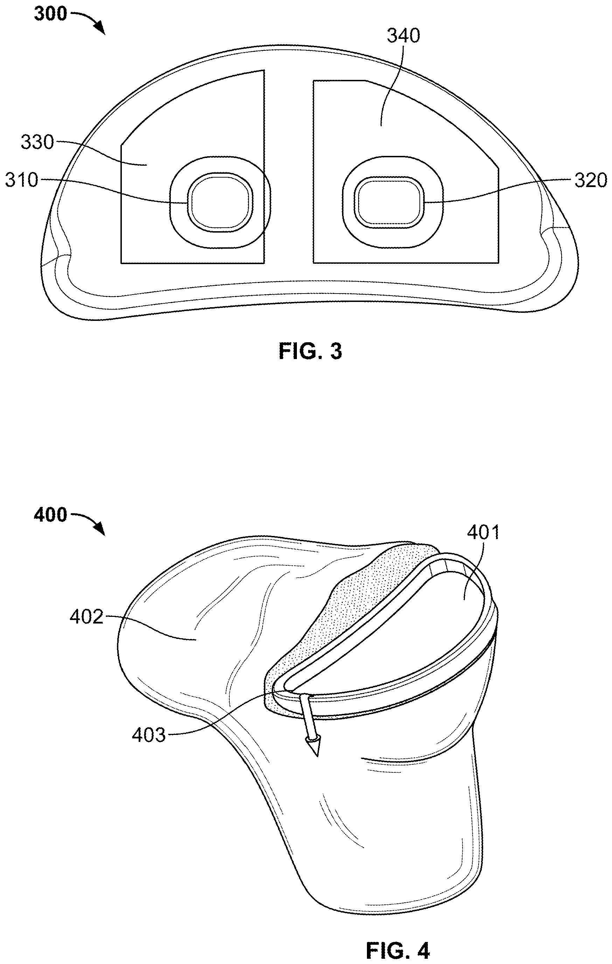

In FIG. 3, a bottom view of a virtual model of an implant is provided. In this case, the implant 300 is a prosthetic tibial baseplate. The design features of the implant may be defined by parameters, which may be adjusted or modified in accordance with the inventive methods described herein. For instance, in this example, fixation features 310 and 320 and/or implant bone contacting geometries 330 and 340 may be parametrically modified. Specific parameters, which may serve as input parameters as described below, may include peg location, depth, angle, curvature, size, press-fit, shape, bone contacting geometry, and/or placement of the degrees of freedom thereof.

In FIG. 4, a probabilistic simulation of a virtual implant on a 3D bone model is provided. In this example of a probabilistic simulation 400, a virtual implant 401 is aligned on the 3D bone model 402 as it would be placed in surgery. Finite element analysis is used to simulate relevant worst case physiological loading scenarios 403. The implant is loaded under a worst case and/or common physiological load 403, and optimal parametric ranges are determined for design features of the articular implant.

Initial image data of at least one joint can be obtained in a variety of ways, including by performing any medical imaging method known in the art, or by obtaining medical image data from a collection and/or database. For example, the image data may be obtained by performing a CT image scan. Additional suitable imaging methods include MRI, Electrical Impedance Tomography ("EIT"), Dual-Energy X-ray Absorptiometry ("DXA" or "DEXA"). X-ray, ultrasound, and nuclear imaging may also be used to obtain the image data, for example. The image data may further comprise a combination of one or more different kinds of image data. For instance, image data may comprise both CT and MRI image data.

The image data may correspond to an individual, a population, or a subpopulation. For instance, the image data may correspond to a bone, including one or more bones of a joint, of the individual for whom the fit of the implant is being optimized. In this case, the parameters of the fixation features are being determined on a patient-specific basis such that the parameters optimize the fit of the implant to the individual. The image data may also be representative of a population, or a subpopulation, for instance corresponding to the representative or average bone properties of a particular population or a specified group of potential patients within the population discriminated by a parameter of interest such as size, gender, morphology, etc. This may be done, for example, by using a database of patient bones. A population may represent a class or sub-class of individuals, for instance members of an age-range, a gender, a class of individuals who suffer or commonly suffer from a particular bone or joint ailment, such as a knee joint ailment, any other suitable population that is relevant to articular implants, or any combination thereof. Statistical shape modeling methods may also be used, for instance, to predict variability of patient morphology and optimize for predicted groups of morphology and density variation. One example of this can be seen in U.S. Patent No. 7,584,080, entitled "Constructing a Statistical Shape Model from Two-Dimensional or Three-Dimensional Data".

Bone property information can be derived from the image data by a variety of methods. There are known methods for calculating or estimating bone properties from the imaging modalities previously described, including CT, X-ray, MRI, DEXA, for example.

By way of example, bone density and elastic modulus can be derived from CT image data by correlating CT brightness to bone density and then to elastic modulus using Hounsfield values. Bone density of both the proximal end of the tibia and the distal end of the femur can be calculated from CT brightness value by the following equations:

A) Proximal Tibia:
Hounsfield unit to density conversion:

$$\rho = 1.14e^{-4} + (9.16e^{-7}) * (CT\#)$$

B) Distal Femur:
Hounsfield unit to density conversion:

$$\rho = 1.39e^{-4} + (1.205e^{-6} *) * (CT\#)$$

where $\rho$ is in g/mm$^3$ and CT# corresponds to the CT number expressed in Hounsfield units.

Further, the elastic modulus of both the proximal end of the tibia and the distal end of the femur can be calculated from the derived density values by the following equations:

A) Proximal Tibia:
Density to modulus conversion:

$$E = (1.2965e^{8}) * (\rho^{1.5}), \ 0 < \rho \leq 0.001 \ g/mm^{3}$$

$$E = (3.790e^{12}) * (\rho^{3}), \ 0.001 < \rho \leq 0.00173 \ g/mm^{3}$$

B) Distal Femur:
Density to modulus conversion:

$$E = (1.283e^{9}) * (\rho^{1.85}), \ 0 < \rho \leq 0.001 \ g/mm^{3}$$

$$E = (3.790e^{12}) * (\rho^{3}), \ 0.001 < \rho \leq 0.00173 \ g/mm^{3}$$

where E is in MPa. The aforementioned examples are only examples of how to derive bone property information from the image data of at least one bone. Similar methods exist and are known in the art related to other forms of image data and other bone properties.

Once local bone properties have been determined from the image data of at least one bone, it is possible to virtually map those bone properties to create a virtual 3D bone model. This can be done using medical imaging or segmentation software. One such software includes Mimics® segmentation software. Local bone properties that may be mapped into a virtual bone model include, but are not limited to, Hounsfield values, derived bone density, elastic modulus, or a combination thereof. Other bone properties relevant to implants may also be derived from the image data and then mapped into a virtual bone model. As should be understood, a virtual bone model, as used herein, includes but is not limited to a three-dimensional ("3D") representation of one or more bones.

By way of example, Hounsfield values may be derived from a CT scan and then mapped to a virtual bone model. In this case, the Hounsfield values are determined from the CT scan and then mapped into a virtual bone model using segmentation software. Then, the mapped Hounsfield values may be converted into local bone density values or elastic modulus using the conversion equations as presented above. The result is a virtual bone model showing areas of decreased and increased bone density and/or elastic modulus.

In an alternative example, bone density can be derived directly from the image data of at least one bone, including one or more bones of a joint, and then mapped directly to a virtual bone model, which may be a 3D model. In this example, bone densities are derived from the image data of at least one bone and then mapped onto a virtual bone model. The same conversion equation between CT brightness and bone density as presented above may also be used. In a further alternative, elastic modulus may be mapped into a virtual bone model, wherein elastic modulus is derived from the image data of at least one bone. In yet a further alternative, the location of brightness values can be exported from a segmentation software, and then mapped using an engineering simulation software such as ANYSY®, for example.

Further, the portion of the virtual bone model that corresponds to the portion of bone that would be resected during surgery may be removed. Thus, corresponding local bone properties mapped to the empty space are discarded.

The design of an implant can be virtually parameterized, for example, defined by one or more parameters such that adjusting or modifying a parameter varies one or more design elements of the implant. Design elements may include all aspects of the design of the implant, including the presence and design of the fixation features. Thus, the number and type of design elements that may be parameterized may include others in addition to those explicitly described herein.

The presence and design of fixation features, which may include pegs, keels, and/or bone contacting geometry, may also be parameterized. Specific parameters may include, for example, any one or more of peg location, peg depth, peg angle, peg curvature, peg size, peg shape, underside peg geometry, and surgical placement degrees of freedom. The bone contacting geometry of the implant may also be a fixation feature that may be parameterized. The design of all fixation features, including bone contacting geometry and the design of all non-peg type fixations features including keels may also be parameterized.

Once the design elements have been parameterized, a value for each parameter can be determined that corresponds to an optimized fit of the articular implant. One such way of determining the optimal values of the design parameters includes finite element analysis, wherein the parameters are varied in the context of various loading scenarios and optimized values for each parameter are determined. Loading scenarios simulate common or worst case physiological loading of the implant virtually placed on a bone.

Initially, the parameters may be given "real-life" or practical ranges or constraints. For instance, there may be design-based or other practical reasons for limiting certain parameters to within a specific range. These ranges may be entered as to limit the parameters to stay within the specific range.

The implant may then be subjected to virtual loading under a worst case or common physiological load, or under a worst case mechanical load, including but not limited to chair rise, stair ascent, and normal level walking, for example.

The simulation may be repeated at each sampled parameter combination and output parameter values are collected. Input parameters may include the parameterized design features, implant geometry and surgical placement of the implant, wherein output parameters may include micromotion, stress transmission, and/or strain. Relationships between input parameters, and output parameters may then be calculated via the simulations. Implant design elements such as fixation feature geometry, and positional limits are analyzed to determine which parameters are most critical to accelerated micromotion, bone stress transmission, implant stresses, etc. Correlation coefficients between each parametric input and response variable are then calculated to determine the effect of each design factor. A contour plot may then be created to determine the optimal range of values for each parameter under different relevant physiological load scenarios. The contour plot, in combination with optional sets of outer practical constraints, may then be used to determine, for example, which combination of inputs (i.e. design features such as peg arrangement, angle, and length) results in an desirable or optimal output (e.g. minimal micro motion). The procedure can thus be used to determine desirable or ideal fixation feature designs, such as geometry and degree of freedom limits for implant placement, based on patient bones under relevant physiological loading. By incorporating probabilistic analysis and patient local property modeling, desired or optimal implant design features can be determined that may maximize fixation effectiveness.

A probabilistic modeling approach may also be used to determine the relationships between the input and output parameters, including Monte Carlo, Bayesian, Advanced Mean Value methods, and other probabilistic modeling methods, for example. Parameter sampling methods including but not limited to Latin hypercube sampling and adaptive sampling techniques may be used to determine the parameter combinations to test in a finite element analysis model. The results from these analyses may be employed to create a response surface to determine desired or optimal parameter combinations.

Correlation coefficients including but not limited to Pearson linear and Spearman rank order correlation coefficients can be calculated to determine the relationships and trends between input and output parameters, along with determining the importance of specific input parameters in optimizing output parameters. The response surface or contour plot created based on input and output parameters could be described with a polynomial, exponential, or other mathematical best fit equations relating the input to the output parameters. As should be understood from the above description, a simulation may use one or more input values and result in one or more output values. Conducting the simulation multiple times for different input values may result in a number of output values from which a relationship may be determined. From a best fit equation of the results, values of design parameters can be calculated to optimize the results of the output parameters.

Once the optimal parametric values have been determined, an implant can be designed and fabricated to reflect the determined desired or optimal parametric values.

Although the disclosure provided herein generally focuses on implants for use in knee replacement surgeries, the invention is not so limited. For example, the concepts disclosed herein may apply to implants for use in other joints, or implants for use in or on bones that are not part of a joint. For example, a hip implant may include an acetabular cup component and/or a femoral stem component. Fixation features of the acetabular cup and/or femoral stem component may be parameterized. A virtual hip model may be created in the same way as described above for a knee joint. A virtual model of the acetabular cup and/or femoral stem may be created and placed on the virtual hip joint. Simulations of the virtual hip implant may be conducted, much as described above, to determine how input parameters of the fixation features of the virtual implant effect output values, such as mircomotion, stress shielding, etc. The input parameters of the acetabular cup, for example, may include the location and number of screw holes in the acetabular cup component, as well as size and thread properties of the corresponding screws, such as lead and pitch. Similarly, input parameters of the femoral stem may include, for example, width, length, curvature, bone contacting geometry, and shape of the femoral stem component.

Still further, the concepts disclosed herein may apply to non-articular implants. For example, desired fixation features of a bone plate may be determined using the methods described herein. Similar to the acetabular cup component described above, a bone plate may include a plurality of through holes that accept screws to fix the bone plate to a bone, the bone plate often intended to span across a fracture in the bone. A virtual model of the bone plate may be created, the virtual bone plate having fixation features in the form of through holes and screws. Input parameters representing these fixation features may include, for example, the number and position of the through holes, as well as the size and thread properties of the corresponding screws. By simulating the fixation of the virtual bone plate to a virtual model of the bone created in the same or a similar way as described above, output parameters including, for example, micro motion, stress shielding, etc. may be related to the input parameters of the fixation features of the bone plate. Based on this analysis, desired fixation features of the bone plate may be determined to reach a desired output, for example minimal micro motion.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

**Claims**

1. A method of designing at least one fixation feature of an implant for a bone, comprising:

   - creating a virtual model of the bone (100, 200, 402);
   - mapping bone property information (101, 102) to the virtual bone, the bone property information derived from image data;
   - creating a virtual model of the implant (300, 401) including at least one virtual fixation feature (310, 320, 330, 340) **characterized by** at least one input parameter;
   - performing a first simulation (400) of an implantation of the virtual implant (300, 401) on the virtual bone (100, 200, 402) with a first value for the at least one input parameter, the first simulation (400) resulting in at least a first value for an output parameter;
   the method being **characterized by**
   - performing a second simulation (400) of the implantation of the virtual implant (300, 401) on the virtual bone (100, 200, 402) with a second value for the at least one input parameter, the second simulation (400) resulting in at least a second value for the output parameter;
   - deriving a relationship between the values of the input parameter and the values of the output parameter, the derived relationship being a mathematical best fit equation; and
   - designing the fixation feature of the implant based on the derived relationship.

2. The method of claim 1, wherein the bone property information (101, 102) is selected from the group of bone density and elastic modulus.

3. The method of claim 1, wherein the step of deriving a relationship between the values of the input parameters and the values of the output parameter includes creating a contour plot or response surface.

4. The method of claim 1, wherein the implant (300, 401) is an articular implant.

5. The method of claim 4, wherein the implant (300, 401) is a knee implant and the fixation feature (310, 320, 330, 340) is a selected from the group consisting of a peg, a keel, and a bone contacting geometry.

6. The method of claim 4, wherein the implant (300, 401) is a knee implant and the input parameter is selected from the group consisting of fixation feature location, fixation feature depth, fixation feature angle, fixation feature curvature, fixation feature size, fixation feature press-fit, fixation feature shape, fixation feature bone contacting geometry, and placement of degrees of freedom of the fixation feature.

7. The method of claim 4, wherein the implant (300, 401) is an acetabular cup component of a hip implant.

8. The method of claim 7, wherein the fixation feature (310, 320, 330, 340) is a one or more through holes in the articular cup component, and the input parameter is selected from the group consisting of the number of through holes in the acetabular cup component and a location of the one or more through holes in the acetabular cup component.

9. The method of claim 7, wherein the fixation feature (310, 320, 330, 340) is one or more screws configured to be inserted through the acetabular cup component, and the input parameter is selected from the group of screw size and thread property.

10. The method of claim 4, wherein the implant (300, 401) is a femoral component of a hip implant and the fixation feature (310, 320, 330, 340) is a femoral stem of the femoral component, and the input parameter is selected from the group consisting of femoral stem width, femoral stem length, femoral stem curvature, femoral stem bone contacting geometry, and femoral stem shape.

11. The method of claim 1, wherein the implant (300, 401) is a non-articular implant.

12. The method of claim 11 wherein the implant (300, 401) is a bone plate and the fixation feature (310, 320, 330, 340) is one or more through holes in the bone plate, and the input parameter is selected from the group consisting of the number of through holes in the bone plate and a location of the one or more through holes in the bone plate.

13. The method of claim 12, wherein the implant (300, 401) is a bone plate and the fixation feature (310, 320, 330, 340) is one or more screws configured to be inserted through the bone plate, and the input parameter is selected from the group of screw size and thread property.

14. The method of claim 1, wherein the output parameter is selected from the group consisting of micro motion, strain transmission, stress transmission, stress shielding.

15. The method of claim 1, wherein the virtual implant (300, 401) is intended for use with cement or other adhesive, and the output parameter is selected from the group of stress transmission to the cement or other adhesive and strain transmission to the cement or other adhesive.

**Patentansprüche**

1. Verfahren zum Entwerfen von mindestens einem Befestigungsmerkmal eines Implantats für einen Knochen, umfassend:

   - Kartieren eines virtuellen Modells des Knochens (100, 200, 402);
   - Abbilden von Knocheneigenschaftsinformationen (101, 102) für den virtuellen Knochen, wobei die Knocheneigenschaftsinformationen von Bilddaten abgeleitet werden;
   - Erzeugen eines virtuellen Modells des Implantats (300, 401), das mindestens ein virtuelles Fixierungsmerkmal (310, 320, 330, 340) beinhaltet, **gekennzeichnet durch** mindestens einen Eingangsparameter;
   - Durchführen einer ersten Simulation (400) einer Implantation des virtuellen Implantats (300, 401) auf dem virtuellen Knochen (100, 200, 402) mit einem ersten Wert für den mindestens einen Eingangsparameter, wobei die erste Simulation (400) zu mindestens einem ersten Wert für einen Ausgabeparameter führt;
   wobei das Verfahren **gekennzeichnet ist durch**
   - Durchführen einer zweiten Simulation (400) der Implantation des virtuellen Implantats (300, 401) auf dem virtuellen Knochen (100, 200, 402) mit einem zweiten Wert für den mindestens einen Eingangsparameter, wobei die zweite Simulation (400) zu mindestens einem zweiten Wert für den Ausgabeparameter führt;

- Ableiten einer Beziehung zwischen den Werten des Eingabeparameters und den Werten des Ausgabeparameters, wobei die abgeleitete Beziehung eine mathematische Gleichung der besten Anpassung ist; und
- Entwerfen des Fixierungsmerkmals des Implantats, basierend auf der abgeleiteten Beziehung.

2. Verfahren nach Anspruch 1, wobei die Knocheneigenschaftsinformation (101, 102) aus der Gruppe Knochendichte und Elastizitätsmodul ausgewählt ist.

3. Verfahren nach Anspruch 1, wobei der Schritt des Ableitens einer Beziehung zwischen den Werten der Eingabeparameter und den Werten des Ausgabeparameters das Erzeugen einer Konturdarstellung oder Antwortoberfläche beinhaltet.

4. Verfahren nach Anspruch 1, wobei das Implantat (300, 401) ein Gelenkimplantat ist.

5. Verfahren nach Anspruch 4, wobei das Implantat (300, 401) ein Knieimplantat ist und das Fixierungsmerkmal (310, 320, 330, 340) aus der Gruppe, bestehend aus einem Stift, einem Kiel und einer Knochenkontaktgeometrie, ausgewählt ist.

6. Verfahren nach Anspruch 4, wobei das Implantat (300, 401) ein Knieimplantat ist und der Eingangsparameter aus der Gruppe, bestehend aus Fixierungsmerkmalsposition, Fixierungsmerkmalstiefe, Fixierungsmerkmalswinkel, Fixierungsmerkmalskrümmung, Fixierungsmerkmalsgröße, Fixierungsmerkmal-Presspassung, Fixierungsmerkmalform, Fixierungsmerkmal-Knochenkontaktgeometrie und Platzierung von Freiheitsgraden des Fixierungsmerkmals, ausgewählt ist.

7. Verfahren nach Anspruch 4, wobei das Implantat (300, 401) eine Hüftgelenkpfannenkomponente eines Hüftimplantats ist.

8. Verfahren nach Anspruch 7, wobei das Fixierungsmerkmal (310, 320, 330, 340) ein oder mehrere Durchgangslöcher in der Gelenkschalenkomponente ist und der Eingangsparameter aus der Gruppe ausgewählt ist, die aus der Anzahl der Durchgangslöcher in der Hüftgelenkpfannenkomponente und einer Stelle des einen oder der mehreren Durchgangslöcher in der Hüftgelenkpfannenkomponente besteht.

9. Verfahren nach Anspruch 7, wobei das Fixierungsmerkmal (310, 320, 330, 340) eine oder mehrere Schrauben ist, die ausgebildet sind, um durch die Hüftgelenkpfannenkomponente eingeführt zu werden, und der Eingabeparameter aus der Gruppe der Schraubengröße und der Gewindeeigenschaft ausgewählt ist.

10. Verfahren nach Anspruch 4, wobei das Implantat (300, 401) eine femorale Komponente eines Hüftimplantats ist und das Fixierungsmerkmal (310, 320, 330, 340) ein femoraler Schaft der femoralen Komponente ist und der Eingangsparameter aus der Gruppe ausgewählt ist, die aus Femurschaftbreite, Femurschaftlänge, Femurschaftkrümmung, Femurschaftknochenkontaktgeometrie und Femurschaftform besteht.

11. Verfahren nach Anspruch 1, wobei das Implantat (300, 401) ein nicht-artikuläres Implantat ist.

12. Verfahren nach Anspruch 11, wobei das Implantat (300, 401) eine Knochenplatte ist und das Fixierungsmerkmal (310, 320, 330, 340) ein oder mehrere Durchgangslöcher in der Knochenplatte ist und der Eingangsparameter aus der Gruppe ausgewählt ist, die aus der Anzahl von Durchgangslöchern in der Knochenplatte und einer Stelle des einen oder der mehreren Durchgangslöcher in der Knochenplatte besteht.

13. Verfahren nach Anspruch 12, wobei das Implantat (300, 401) eine Knochenplatte ist und das Fixierungsmerkmal (310, 320, 330, 340) eine oder mehrere Schrauben ist, die ausgebildet sind, um durch die Knochenplatte eingeführt zu werden und der Eingabeparameter aus der Gruppe Schraubengröße und Gewindeeigenschaft ausgewählt ist.

14. Verfahren nach Anspruch 1, wobei der Ausgangsparameter aus der Gruppe, bestehend aus Mikrobewegung, Dehnungsübertragung, Belastungsübertragung, Stressabschirmung, ausgewählt ist.

15. Verfahren nach Anspruch 1, wobei das virtuelle Implantat (300, 401) zur Verwendung mit Zement oder einem anderen Klebstoff vorgesehen ist und der Ausgabeparameter aus der Gruppe der Spannungsübertragung auf den Zement oder anderen Klebstoff und der Dehnungsübertragung auf den Zement oder anderen Klebstoff ausgewählt ist.

**Revendications**

1. Procédé de conception d'au moins une caractéristique de fixation d'un implant pour un os, comprenant :

   - créer un modèle virtuel de l'os (100, 200, 402) ;
   - cartographier une information de propriété osseuse (101, 102) sur l'os virtuel, l'information de propriété osseuse étant dérivée de données d'images ;
   - créer un modèle virtuel de l'implant (300, 401) incluant au moins une caractéristique de fixation virtuelle (310, 320, 330, 340) **caractérisé par** au moins un paramètre d'entrée ;
   - réaliser une première simulation (400) d'une implantation de l'implant virtuel (300, 401) sur l'os virtuel (100, 200, 402) avec une première valeur pour l'au moins un paramètre d'entrée, la première simulation (400) résultant en au moins une première valeur pour un paramètre de sortie ;
   le procédé étant **caractérisé par**
   - réaliser une deuxième simulation (400) de l'implantation de l'implant virtuel (300, 401) sur l'os virtuel (100, 200, 402) avec une deuxième valeur pour l'au moins un paramètre d'entrée, la deuxième simulation (400) résultant en au moins une deuxième valeur pour le paramètre de sortie ;
   - dériver une relation entre les valeurs du paramètre d'entrée et les valeurs du paramètre de sortie, la relation dérivée étant une équation de meilleur ajustement mathématique ; et
   - concevoir la caractéristique de fixation de l'implant sur la base de la relation dérivée.

2. Procédé selon la revendication 1, dans lequel l'information de propriété osseuse (101, 102) est sélectionnée dans le groupe de la densité osseuse et du module élastique.

3. Procédé selon la revendication 1, dans lequel l'étape de dériver une relation entre les valeurs des paramètres d'entrées et les valeurs du paramètre de sortie inclut la création d'un tracé de contour ou d'une surface de réponse.

4. Procédé selon la revendication 1, dans lequel l'implant (300, 401) est un implant articulaire.

5. Procédé selon la revendication 4, dans lequel l'implant (300, 401) est un implant de genou et la caractéristique de fixation (310, 320, 330, 340) est sélectionnée dans le groupe constitué d'une cheville, d'une quille, et d'une géométrie au contact de l'os.

6. Procédé selon la revendication 4, dans lequel l'implant (300, 401) est un implant de genou et le paramètre d'entrée est sélectionné dans le groupe constitué d'un emplacement de la caractéristique de fixation, d'une profondeur de la caractéristique de fixation, d'un angle de la caractéristique de fixation, d'une courbure de la caractéristique de fixation, d'une taille de la caractéristique de fixation, d'un ajustement serré de la caractéristique de fixation, d'une forme de la caractéristique de fixation, d'une géométrie au contact de l'os de la caractéristique de fixation, et d'un placement des degrés de liberté de la caractéristique de fixation.

7. Procédé selon la revendication 4, dans lequel l'implant (300, 401) est composant de coupe acétabulaire d'un implant de hanche.

8. Procédé selon la revendication 7, dans lequel la caractéristique de fixation (310, 320, 330, 340) est un ou plusieurs trous traversants dans le composant de coupe articulaire, et le paramètre d'entrée est sélectionné dans le groupe constitué du nombre de trous traversants dans le composant de coupe acétabulaire et d'un emplacement de l'un ou plusieurs trous traversants dans le composant de coupe acétabulaire.

9. Procédé selon la revendication 7, dans lequel la caractéristique de fixation (310, 320, 330, 340) est une ou plusieurs vis configurées pour être insérées à travers le composant de coupe acétabulaire, et le paramètre d'entrée est sélectionné dans le groupe d'une taille de vis et d'une propriété de filetage.

10. Procédé selon la revendication 4, dans lequel l'implant (300, 401) est un composant fémoral d'un implant de hanche et la caractéristique de fixation (310, 320, 330, 340) est une tige fémorale du composant fémoral, et le paramètre d'entrée est sélectionné dans le groupe constitué de la largeur de la tige fémorale, de la longueur de la tige fémorale, de la courbure de la tige fémorale, de la géométrie au contact de l'os de la tige fémorale, et de la forme de la tige fémorale.

11. Procédé selon la revendication 1, dans lequel l'implant (300, 401) est un implant non-articulaire.

**12.** Procédé selon la revendication 11 dans lequel l'implant (300, 401) est une plaque d'os et la caractéristique de fixation (310, 320, 330, 340) est un ou plusieurs trous traversants dans la plaque d'os, et le paramètre d'entrée est sélectionné dans le groupe constitué du nombre de trous traversants dans la plaque d'os et d'un emplacement de l'un ou plusieurs trous traversants dans la plaque d'os.

**13.** Procédé selon la revendication 12, dans lequel l'implant (300, 401) est une plaque d'os et la caractéristique de fixation (310, 320, 330, 340) est une ou plusieurs vis configurées pour être insérées à travers la plaque d'os, et le paramètre d'entrée est sélectionné dans le groupe d'une taille de vis et d'une propriété de filetage.

**14.** Procédé selon la revendication 1, dans lequel le paramètre de sortie est sélectionné dans le groupe constitué d'un micro déplacement, d'une transmission de contrainte, d'une transmission de stress, d'une protection contre le stress.

**15.** Procédé selon la revendication 1, dans lequel l'implant virtuel (300, 401) est destiné à une utilisation avec du ciment ou un autre adhésif, et le paramètre de sortie est sélectionné dans le groupe de transmission de stress au ciment ou à un autre adhésif et de transmission de contrainte au ciment ou à un autre adhésif.

**FIG. 1**

**FIG. 2**

300

340

330

310

320

**FIG. 3**

400

401

402

403

**FIG. 4**

**EP 3 062 746 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20120092451 A
- US 7584080 B